# EUROPÄISCHE PATENTANMELDUNG

(11) **EP 2 005 937 A1**
(43) Veröffentlichungstag der Anmeldung: **24.12.2008**
(21) Anmeldenummer: 08009680.3
(22) Anmeldetag: 28.05.2008
(51) Int. Cl.: A61K 8/06, A61K 8/22, A61K 8/37, A61K 8/58, A61Q 19/00

(54) **Langsam Sauerstoff freisetzende kosmetische Zubereitungen**

(30) Priorität: 14.06.2007 DE 102007028026
(71) Anmelder: Beiersdorf Aktiengesellschaft, 20245 Hamburg (DE)
(72) Erfinder: Treu, Jens, Dr., 22844 Norderstedt (DE); Kolbe, Ludger, Dr., 21255 Dohren (DE); Fey, Sven, Dr., 22397 Hamburg (DE); Rathsack, Jessica, 21614 Buxtehude (DE); Möllgaard, Svenja Lena, Dr., 22301 Hamburg (DE); Peters, Nils, Dr., 22965 Todendorf (DE)
(74) Vertreter: Wilke, Jochen

(57) **Zusammenfassung**

Kosmetische O/W-Emulsionen mit einem Gehalt an molekularem Sauerstoff, die einen Gehalt von mehr als 5 Gew-% an nichtflüchtige Ölen und mehr als 4 Gew- % flüchtige Öle sowie weniger als 8 Gew.-% an einer oder mehreren Feuchtigkeit spendenden Substanzen ("Moisturizer") aufweisen.

## Beschreibung

Die Erfindung betrifft schnell Sauerstoff freisetzende kosmetische Zubereitungen.

Gashaltige kosmetische Zubereitungen sind an sich bekannt und bereits in zahlreichen Patenten beschrieben worden. Als Gase werden beispielsweise Sauerstoff, fluorierte Gase, Kohlendioxid, Luft, Stickstoff und dergleichen verwendet.

Aus dem Stand der Technik sind kosmetische Zubereitungen auf Basis von O/W-Emulsionen bekannt, die Sauerstoff enthalten.

Die WO 02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung / Prävention von Sauerstoffmangelzuständen der Haut.

Die WO 02/05754 offenbart O/W-Emulsionen, die als einen Sauerstofftransport durch die Lipidschicht der Haut adäquat gewährleisten sollen.

In der US 5851544 wird beschrieben, dass Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5851544 ist es daher, den Sauerstoffgehalt der Haut langfristig zu steigern bzw. Sauerstoffmangelzustände in der Haut zu beheben.

In der DE 10333710 werden kosmetische, dermatologische oder pharmazeutische Zubereitungen auf Basis von Lipiden und/oder Lipid/Wachs-Gemischen, welche ein Gas bzw. ein Gasgemisch enthalten, beschrieben.

Die DE 10342449, DE 102005011498, DE 102005011457 und DE 102206011459 offenbaren kosmetische Zubereitungen enthaltend molekularen Sauerstoff. Auch hierin werden bevorzugt O/W-Emulsionen beschrieben. Die Herstellung der sauerstoffhaltigen O/W-Emulsionen erfolgt indem eine Vordispergierung des anorganischen Gelbildners und Quellung des Hydrokolloides unter Rühren in der Wasserphase stattfindet. Eine Vereinigung der auf 75 °C aufgeheizten Fettphase mit der auf 70°C aufgeheizten Wasserphase und Zugabe der partikulären hydrophoben, hydrophobisierten Festkörpersubstanzen unter Rühren. Es erfolgt dann Homogenisierung bei 65 °C und 45 min Rühren unter Vakuum und Kühlung. Die Zugabe der Additive erfolgt bei 30 °C, die Homogenisierung bei 27°C. Anschließend wird die Begasung der Emulsion bei 7-8 bar mit Sauerstoff durchgeführt.

Die Herstellung sauerstoffhaltiger kosmetischer Zubereitungen und insbesondere der Schritt der Sauerstoffzufuhr erfolgt in allen aufgeführten Druckschriften des Standes der Technik dabei unter Kühlung, bei Raumtemperatur bzw. bei maximal 30°C.

Die Wirkung von Sauerstoff auf die Haut hängt bekanntermaßen stark vom Zeitraum ab, den die Haut intensiven Kontakt mit dem Gas hat. Die Freigabe von Sauerstoff aus begasten Produkten ist somit ein entscheidener Faktor für die positive Wirkung des Gases. Die Sauerstoff-Freigabe aus kosmetischen Produkten kann nach dem bisherigem Stand der Technik nicht beeinflusst werden.

Es wurde überraschend festgestellt, daß kosmetische O/W-Emulsionen mit einem Gehalt an molekularem Sauerstoff, die einen Gehalt von mehr als 5 Gew-% an nichtflüchtige Ölen und mehr als 4 Gew- % flüchtige Öle sowie weniger als 8 Gew.-% an einem oder mehreren Feuchtigkeit spendenden Substanzen ("Moisturizer") aufweisen, langsamer und gleichmäßiger Sauerstoff an die Haut abgeben, als der Stand der Technik vermuten ließ.

Als nichtflüchtig bezeichnet man ein Öl, wenn es für mehrere Stunden auf der Haut oder den Lippen bleibt. Es hat im allgemeinen einen Dampfdruck bei Raumtemperatur von kleiner als 0,02 mm Hg (2,66 Pa).

Solche Öle können vorteilhaft gewählt werden aus der Gruppe der Mineralöle, Avocadoöl, Babassuöl, Octyldodecanol, Mittelkettige Triglyceride, Dicaprylylether, Isopropylpalmitat, Isostearyl Isostearat, dick- und dünnflüssiges Paraffin, Vaseline, Jojobaöl, Macadamiaöl, Mandelöl, Nachtkerzenöl, Teebaumöl, hydrogenierte Cocoglyceride, Ringelblumenöl, Octyldodecyl Myristat, Butylene Glykol Dicaprylat/Dicaprat, Hydrogenated Vegetable Oil, Shea Butter, Dicaprylyl Carbonat, Ethylhexyl Stearat, Isopropyl Isostearat, Cetearyl Isononanoat, Isopropyl Stearat, C12-15 Alkyl Benzoat, Traubenkernöl, hydrogeniertes Polyisobuten, Dimethicon, Cetyl Dimethicon, Dimethiconol.

Ein flüchtiges Öl verdampft innerhalb von etwa einer Stunde von der Haut oder den Lippen. Es hat im allgemeinen einen Dampfdruck bei Raumtemperatur, der zwischen 0,02 mmHg und 300 mm Hg (13 Pa -12000 Pa), bevorzugt zwischen 0,1 mmHg und 90 mm Hg (2,66 Pa-40000 Pa) liegt.

Als flüchtige Ölkomponente können vorteilhaft flüchtige Siliconöle; insbesondere Cyclomethicone gewählt werden.

Es wird bevorzugt, die Silicone der erfindungsgemäßen Zubereitungen aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

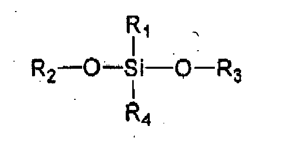

Als erfindungsgemäß vorteilhaft einzusetzenden linearen Silicone mit mehreren Siloxyleinheiten werden im allgemeinen durch Strukturelemente charakterisiert wie folgt: 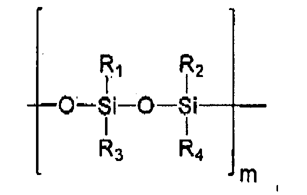 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist) m kann dabei Werte von 2 - 200.000 annehmen.

Erfindungsgemäß vorteilhaft einzusetzende cyclische Silicone werden im allgemeinen durch Strukturelemente charakterisiert, wie folgt 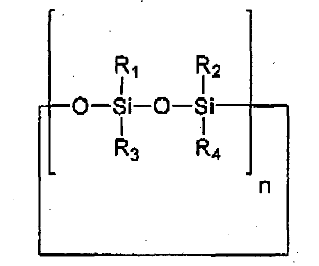 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist) n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Siliconöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Erfindungsgemäß vorteilhaft sind solche Zubereitungen, die einen Gehalt von höchstens 12 Gew-% an nichtflüchtigen Ölen und höchstens 10 Gew- % an flüchtigen Ölen aufweisen.

Als feuchtigkeitsspendende Agentien können vorteilhaft Polyole Anwendung finden, insbesondere Glycerin, Ethylhexylhexylglycerin, 1,2-Hexandiol, 1,2-Pentandiol, 1,2-Octandiol, Methylpropandiol, Butylenglykol aber auch Harnstoff, Milchsäure, Citronensäure, Sorbitol, Aminosären, Glucosamen, Kreatin, Kreatinin, oder Carnitin.

Vorteilhaft enthalten die erfindungsgemäßen Zubereitungen 5-30 Volumen-% Sauerstoff, vorzugsweise 10 - 20 Volumen-%, insbesondere bevorzugt 12-17 Volumen-%

Vorteilhaft enthalten die erfindungsgemäßen Zubereitungen 0,1 -5 Gew-% an Emulgator A, vorzugsweise 0,5 - 2,5 Gew-% an Emulgator A, insbesondere bevorzugt 1- 2 Gew-% an Emulgator A.

Vorteilhaft enthalten die erfindungsgemäßen Zubereitungen 0,1 - 5 Gew-% an Emulgator B, vorzugsweise 0,5 - 2,5 Gew-% an Emulgator B, insbesondere bevorzugt 1- 2Gew-% an Emulgator B.

Vorteilhaft enthalten die erfindungsgemäßen Zubereitungen 0,1 - 5 Gew-% an einer oder mehreren UV-Licht absorbierenden Substanzen, vorzugsweise 0,5 - 2,5 Gew-% an an einer oder mehreren UV-Licht absorbierenden Substanzen, insbesondere bevorzugt 1-2 Gew-% an einer oder mehreren UV-Licht absorbierenden Substanzen.

Die erfindungsgemäße kosmetische, oder dermatologische Zubereitung, umfassend molekularen Sauerstoff ist dadurch charakterisiert, dass sie erhältlich ist durch übliches Mischen und Herstellen einer O/W-Emulsion und anschließendem Begasen der Zubereitung mit gasförmigen Sauerstoff und/oder sauerstoffhaltigen Gasen.

Die erfindungsgemäße kosmetische oder dermatologische Zubereitung auf Basis einer Emulsion, insbesondere einer O/W-Emulsion, umfassend molekularen Sauerstoff ist dadurch charakterisiert, dass sie erhältlich ist durch übliches Mischen und Herstellen einer Emulsion und anschließendem Begasen der Zubereitung mit gasförmigen Sauerstoff und/oder sauerstoffhaltigen Gasen. Dabei wird die Zubereitung bei Raumtemperatur (ca. 20 bis 25 °C) in den Mischer eingespeist und anschließend begast.

Die kosmetischen und dermatologischen Zubereitungen gemäß der Erfindung können kosmetische Hilfsstoffe enthalten, wie sie üblicherweise in solchen Zubereitungen verwendet werden, z. B. Emulgatoren, Pigmente, Konservierungsmittel, Konservierungshelfer, Komplexbildner, Bakterizide, Parfüme, Substanzen zum Steigern des Schäumens, Farbstoffe, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Durch die Verwendung von Sauerstoff bzw. kosmetischer oder topischer dermatologischer Zubereitungen mit einem wirksamen Gehalt an Sauerstoff im Sinne der vorliegenden Erfindung wird die Haut überraschend besser gegen Alterungsprozesse, Struktur- und/oder oxidative Schäden geschützt als dies mit Zubereitungen des Standes der Technik möglich wäre. Ferner wird durch die erfindungsgemäße Verwendung die Wiederherstellung des gesunden Hautzustands im Fall einer bereits bestehenden Störung und Schädigung der normalen Hauteigenschaften begünstigt.

Unter "gestörtem Erscheinungsbild der Haut" sind im Sinne der vorliegenden Erfindung insbesondere die folgenden Phänomene zu verstehen:
■ Falten und/oder Fältchen,
■ Haut- und/oder Gewebeerschlaffung,
■ Störungen der Regeneration der Haut und der Hautanhangsgebilde,
■ Durchblutungsstörungen der Haut,
■ Altersflecken, Pigmentstörungen und sogenannter "uneven skin tone"
■ sensible Haut,
■ Juckreiz,
■ Stressempfindlichkeit der Haut,
■ entzündliche und empfindliche Hautzustände,
■ trockene Hautzustände,
■ atopisches Ekzem,
■ Psoriasis
■ Unregelmäßige bzw. zunehmende Porengröße

Ferner werden bezogen auf die Hautanhangsgebilde (Haare, Nägel) insbesondere die folgenden (Alterungs-) Erscheinungen ("gestörte Erscheinungsbilder der Haare") durch die erfindungsgemäße Verwendung positiv beeinflußt:
- Wachstumsverlangsamung,
- Deformation,
- Spliss,
- Rissigkeit,
- vorzeitiger Verlust,
- Struktur und Farbveränderung.

Die kosmetischen oder dermatologischen Mittel gemäß der Erfindung können vorteilhaft in Form von schäumbaren Zubereitungen vorliegen, welche beispielsweise aus Aerosolbehältem entnommen und dabei aufgeschäumt werden. Erfindungsgemäß vorteilhafte Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher Bauart ausgestattet sein, die die Entnahme des Inhalts als Schaum ermöglichen.

Die kosmetischen oder dermatologischen Formulierungen im Sinne der vorliegenden Erfindung können wie üblich zusammengesetzt sein und dem kosmetischen oder dermatologischen Lichtschutz, ferner zur Behandlung, Pflege und Reinigung der Haut, der Lippen, sowie von Hautanhangsgebilden (Nägel und/oder Haare) und als Schminkprodukt in der dekorativen Kosmetik dienen.

Ebenso wie Emulsionen von flüssiger und fester Konsistenz als kosmetische Reinigungslotionen bzw. Reinigungscremes Verwendung finden, können auch die Zubereitungen im Sinne der vorliegenden Erfindung "Reinigungsschäume" darstellen, welche beispielsweise zum Entfernen von Schminken und/oder Make-up oder als milder Wasch- und Duschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| **O/W-Emulsionen** | **1** |
|---|---|
| Glyceryl Stearat Citrat | 2 |
| Avocadoöl | 1 |
| C12-15 Alkyl Benzoat | 2 |
| Macadamiaöl | 1 |
| Isopropyl Stearat | 2 |
| Dicaprylyl Carbonat | 2 |
| Cyclomethicon | 8 |
| Myristyl Myristat | 1 |
| Cetylalkohol | 1,5 |
| Zitronensäure | 0,03 |
| Butylmethoxydibenzoylmethan | 2 |
| Octocrylen | 5 |
| Phenylbenzimidazolsulfonsäure | 1 |
| Titandioxid | 1 |
| EDTA | 2 |
| BHT | 0,1 |
| 1,2-Hexandiol | 0,2 |
| 1,2-Pentandiol | 0,2 |
| Glycerin | 4 |
| Parfum | 0,3 |
| Carbomer | 0,7 |
| Kreatin | 0,01 |
| Vitamin E Acetat | 1,5 |
| Nylon-12 | 2 |
| Phenoxyethanol | 0,5 |
| Propylparaben | 0,2 |
| Methylparaben | 0,2 |
| Natriumhydroxid | q.s. |
| Wasser | ad 100 |

| | |
|---|---|
| Die Emulsion wird nach Herstellung mit 18% Sauerstoff begast. | |

| **O/W-Emulsionen** | **2** |
|---|---|
| Polyglyceryl-3 Methyl Glucose Distearat | 4 |
| C12-15 Alkylbenzoat | 2 |
| Dimethicon | 2 |
| Dicaprylylcarbonat | 1 |
| Butylene Glycol Dicaprylate/Dicaprate | 2 |
| Mandelöl | 1 |
| Nachtkerzenöl | 1,5 |
| Cyclomethicon | 6 |
| Myristyl Myristat | 1 |
| Cetylalkohol | 2 |
| Zitronensäure | 0,05 |
| Butylmethoxydibenzoylmethan | 0,5 |
| Ethylhexyl Methoxycinnamat | 3 |
| EDTA | 1 |
| BHT | 0,05 |
| 1,2-Octandiol | 0,3 |
| Methylpropandiol | 2 |
| Glycerin | 2,2 |
| Parfum | 0,4. |
| Xanthangummi | 0,4 |
| Vitamin E Acetat | 1 |
| Phenoxyethanol | 0,4 |
| Ethylparaben | 0,1 |
| Propylparaben | 0,1 |
| Methylparaben | 0,2 |
| Natriumhydroxid | 0,2. |
| Wasser | ad 100 |

| | |
|---|---|
| Die Emulsion wird nach Herstellung mit 25% Sauerstoff begast. | |

| **O/W-Emulsionen** | **3** |
|---|---|
| Stearinsäure | 3 |
| Cyclomethicon | 5,5 |
| Dimethicon | 4 |
| Butylenglycol Dicaprylat/Dicaprat | 3 |
| Caprylsäure/Caprinsäure Triglyceride | 2 |
| Glycerylstearat | 1 |
| Stearylalkohol | 2 |
| Zitronensäure | 0,01 |
| Bis-Ethylhexyloxyphenolmethoxyphenyltriazin | 1 |
| Ethylhexyltriazon | 1 |
| Octocrylen | 2 |
| Dinatriumphenyldibenzimidazoltetrasulfonat | 4 |
| Zinkoxid | 1 |
| EDTA | 1 |
| Glycerin | 3 |
| Ethylhexylglycerin | 2 |
| Parfum | 0,1 |
| Acrylates/C10-30 Alkyl Acrylate Crosspolymer | 0,4 |
| Panthenol | 1 |
| Ubichinon | 0,01 |
| Vitamin E Acetat. | 0,2 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

| | |
|---|---|
| Die Emulsion wird nach Herstellung mit 15% Sauerstoff begast. | |

| **O/W-Emulsionen** | **4** |
|---|---|
| Glyceryl Stearat Citrat | 2,5 |
| Cyclomethicon | 9 |
| C12-15 Alkylbenzoat | 2 |
| Dimethicon | 2 |
| Hydrogenierte Cocoglyceride | 2 |
| Traubenkemöl | 1 |
| Myristyl Myristat | 0,5 |
| Cetylstearylalkohol | 2 |
| Zitronensäure | 0,05 |
| Butylmethoxydibenzoylmethan | 4 |
| Titandioxid | 1 |
| Zinkoxid | 1 |
| Ethylhexylsalicylat | 2 |
| Homosalat | 2 |
| EDTA | 1 |
| BHT | 0,1 |
| Ethylhexylglycerin | 2 |
| Glycerin | 2,8 |
| Parfum | q.s. |
| Acrylates/C10-30 Alkyl Acrylate Crosspolymer | 0,5 |
| Kreatin | 0,1 |
| Ubichinon | 0,01 |
| Tapiocastärke | 1 |
| Phenoxyethanol | 0,5 |
| Propylparaben | 0,2 |
| Methylparaben | 0,2 |
| Natriumhydroxid | 0,15 |
| Wasser | ad 100 |

| | |
|---|---|
| Die Emulsion wird nach Herstellung mit 22% Sauerstoff begast. | |

| **O/W-Emulsionen** | **5** |
|---|---|
| PEG-40 Stearat | 3 |
| Dicaprylylether | 2 |
| Cyclomethicon | 6,5 |
| Hydrogenierte Cocoglyceride | 2 |
| Cetyldimeticon | 3 |
| Caprylsäure/Caprinsäure Triglyceride | 2 |
| Octyldodecanol | 2 |
| Cetylalkohol | 2 |
| Butylmethoxydibenzoylmethan | 0,5 |
| Ethylhexylmethoxycinnamat | 3 |
| EDTA | 1 |
| BHT | 0,05 |
| 1,2-Octandiol | 0,25 |
| Glycerin | 5 |
| Parfum | 0,3. |
| Xanthangummi | 0,6 |
| Panthenol | 1,5 |
| Vitamin E Acetat | 0,5 |
| Nylon-12 | 2 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

| | |
|---|---|
| Die Emulsion wird nach Herstellung mit 21% Sauerstoff begast. | |

| **O/W-Emulsionen** | **6** |
|---|---|
| Glyceryl Stearat Citrat | 3 |
| Butylenglycol Dicaprylat/Dicaprat | 3 |
| Dicaprylylcarbonat | 2 |
| Babassuöl | 0,8 |
| Cetyldimeticon | 1 |
| Isopropyl Palmitat | 1 |
| Isohexadecan | 6 |
| Cetylstearylalkohol | 1 |
| Stearylalkohol | 1 |
| Zitronensäure | 0,1 |
| Bis-Ethylhexyloxyphenolmethoxyphenyltriazin | 0,5 |
| Octocrylen | 5 |
| Dinatriumphenyldibenzimidazoltetrasulfonat | 3 |
| EDTA | 2 |
| BHT | 0,1 |
| Methylpropandiol | 2 |
| 1,2-Pentandiol | 1 |
| Glycerin | 2 |
| Parfum | q.s. |
| Carbomer | 0,8 |
| Phenoxyethanol | 0,4 |
| Ethylparaben | 0,1 |
| Propylparaben | 0,1 |
| Methylparaben | 0,2 |
| Natriumhydroxid | 0,1 |
| Wasser | ad 100 |

| | |
|---|---|
| Die Emulsion wird nach Herstellung mit 12% Sauerstoff begast. | |

| **O/W-Emulsionen** | **7** |
|---|---|
| Sucrose Polystearat | 3 |
| Natrium Stearoylglutamat | 0,5 |
| C12-15 Alkylbenzoat | 2,5 |
| Dimethicon | 4,5 |
| Cyclomethicon | 11 |
| Cetylalkohol | 3 |
| Butylmethoxydibenzoylmethan | 2 |
| Ethylhexylmethoxycinnamat | 2 |
| Ethylhexyltriazon | 2 |
| Ethylhexylsalicylat | 2 |
| EDTA | 1 |
| BHT | 0,1 |
| 1,2-Pentandiol | 0,25 |
| 1,2-Hexandiol | 0,5 |
| Glycerin | 4 |
| Parfum | q.s. |
| Carbomer | 0,6 |
| Folsäure | 0,01 |
| Silica | 1 |
| Phenoxyethanol | 0,5 |
| Propylparaben | 0,2 |
| Methylparaben | 0,2 |
| Natriumhydroxid | 0,15 |
| Wasser | ad 100 |

| | |
|---|---|
| Die Emulsion wird nach Herstellung mit 8% Sauerstoff begast. | |

| **O/W-Emulsionen** | **8** |
|---|---|
| Potassium Cetyl Phosphat + Hydrogenierte Palmglyceride | 4 |
| Cyclomethicon | 5,5 |
| Hydrogenierte Cocosfettsäureglyceride | 2 |
| Cetyldimeticon | 3 |
| Caprylsäure/Caprinsäuretriglyceride | 1 |
| Butylene Glycol Dicaprylate/Dicaprat | 2 |
| Cetylalkohol | 1 |
| Stearylalkohol | 1 |
| Zitronensäure | 0,1 |
| Bis-Ethylhexyloxyphenolmethoxyphenyltriazin | 2 |
| Octocrylen | 1 |
| Titandioxid | 2 |
| 1,2-Hexandiol | 0,2 |
| Glycerin | 4 |
| Parfum | 0,4 |
| Cl 77492 + Cl 77491 + Cl 77499 | 0,1 |
| Cl 77891 | 2 |
| Vitamin E Acetat | 1 |
| Silica + Cl 77891 + Cl 77491 | 1 |
| Phenoxyethanol | 0,4 |
| Ethylparaben | 0,1 |
| Propylparaben | 0,1 |
| Methylparaben | 0,2 |
| Natriumhydroxid | 0,2. |
| Wasser | ad 100 |

| | |
|---|---|
| Die Emulsion wird nach Herstellung mit 15% Sauerstoff begast. | |

| **O/W-Emulsionen** | **9** |
|---|---|
| Glyceryl Stearat Citrat | 3 |
| C12-15 Alkylbenzoat | 4 |
| Hydrogenierte Cocosfettsäureglyceride | 1 |
| Jojobaöl | 4 |
| Cetylalkohol | 1 |
| Cetylstearylalkohol | 1 |
| Stearylalkohol | 1 |
| Cyclomethicon | 6 |
| BHT | 0,2 |
| Glycerin | 1 |
| Parfum | q.s. |
| Xanthangummi | 0,8 |
| DHA | 1 |
| Cl 77891 + Mica + Silika | 1 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

| | |
|---|---|
| Die Emulsion wird nach Herstellung mit 28% Sauerstoff begast. | |

## Patentansprüche

1. Kosmetische O/W-Emulsionen mit einem Gehalt an molekularem Sauerstoff, die einen Gehalt von mehr als 5 Gew-% an nichtflüchtige Ölen und mehr als 4 Gew- % flüchtige Öle sowie weniger als 8 Gew.-% an einer oder mehreren Feuchtigkeit spendenden Substanzen ("Moisturizer") aufweisen.

2. Emulsionen nach Anspruch 1, enthaltend 5 - 30 Volumen-% Sauerstoff, vorzugsweise 10 - 20 Volumen-%, insbesondere bevorzugt 12 - 17 Volumen-%

3. Emulsionen nach Anspruch 1 oder 2, bei denen nichtflüchtige Öl einen Dampfdruck bei Raumtemperatur von kleiner als 0,02 mm Hg (2,66 Pa) aufweist.

4. Emulsionen nach einem der vorstehenden Ansprüche, bei denen das nichtflüchtige Öl oder die nichtflüchtigen Öle aus der Gruppe Mineralöle, Avocadoöl, Babassuöl, Octyldodecanol, Caprylsäure/Caprinsäure Triglyceride, Dicaprylylether, Isopropylpalmitat, Isostearyl Isostearat, dick- und dünnflüssiges Paraffin, Vaseline, Jojobaöl, Macadamiaöl, Mandelöl, Nachtkerzenöl, Teebaumöl, hydrogenierte Cocoglyceride, Ringelblumenöl, Octyldodecyl Myristat, Butylene Glykol Dicapry-lat/Dicaprat, Hydrogenated Vegetable Oil, Shea Butter, Dicaprylyl Carbonat, Ethylhexyl Stearat, Isopropyl Isostearat, Cetearyl Isononanoat, Isopropyl Stearat, C12-15 Alkyl Benzoat, Traubenkernöl, hydrogeniertes Polyisobuten, Dimethicon, Cetyl Dimethicon, Dimethiconol gewählt wird oder werden.

5. Emulsionen nach einem der vorstehenden Ansprüche, bei denen das flüchtige ÖI einen Dampfdruck bei Raumtemperatur aufweist, der zwischen 0,02 mmHg und 300 mm Hg (13 Pa - 12000 Pa), bevorzugt zwischen 0,1 mmHg und 90 mm Hg (2,66 Pa - 40000 Pa) liegt.

6. Emulsionen nach einem der vorstehenden Ansprüche, bei denen das nichtflüchtige Öl oder die nichtflüchtigen Öle aus der Gruppe der flüchtigen Siliconöle, insbesondere Cyclomethicone, gewählt werden.

7. Emulsionen nach einem der vorstehenden Ansprüche, bei denen Feuchtigkeit spendenden Substanz oder Substanzen gewählt wird oder werden aus der Gruppe der Polyole, insbesondere Glycerin, Ethylhexylhexylglycerin, 1,2-Hexandiol, 1,2-Pentandiol, 1,2-Octandiol, Methylpropandiol, Butylenglykol aber auch Harnstoff. Milchsäure, Citronensäure, Sorbitol, Aminosäuren, Glucosamen, Kreatin, Kreatinin, oder Carnitin.

8. Emulsionen nach einem der vorstehenden Ansprüche, die einen Gehalt von höchstens 12 Gew-% an nichtflüchtigen Ölen und höchstens 10 Gew- % an flüchtigen Ölen aufweisen.
